# EUROPEAN PATENT APPLICATION

(11) **EP 3 705 117 A1**
(43) Date of publication of application: **09.09.2020**
(21) Application number: 19382161.8
(22) Date of filing: 05.03.2019
(51) Int. Cl.: A61K 9/16, A61K 9/20, A61K 31/4045

(54) **MELATONIN HAVING IMPROVED WATER SOLUBILITY, ITS PREPARATION AND USES THEREOF**

(71) Applicant: Tradichem Industrial Services, S.L., 28013 Madrid (ES)
(72) Inventor: MARAÑÓN MAROTO, José Ángel, 28240 HOYO DE MANZANARES (ES); MORENO PUENTE, Patricia, 28007 MADRID (ES); LOZANO MARTÍN, Cristina, 28251 RIVAS VACIAMADRID (ES); MANCHA AVELLÁN, María, 28006 MADRID (ES)
(74) Representative: Sugrañes Patentes y Marcas

(57) **Abstract**

The invention relates to a process for preparing substantially pure melatonin having improved water solubility comprising subjecting melatonin to a roller compaction process and also relates to the compacted melatonin obtainable with such process. The process of the invention allows obtaining melatonin with improved water solubility, which is advantageous for the preparation of pharmaceutical compositions and for their use in therapy. Therefore, the invention also relates to the use of compacted melatonin for preparing pharmaceutical compositions, to pharmaceutical compositions comprising compacted melatonin as active ingredient, and to pharmaceutical compositions comprising compacted melatonin for use in therapy.

## Description

### Technical field

The present invention relates to melatonin having improved solubility in water, which is particularly suitable for the manufacture of pharmaceutical dosage forms and for use in therapy.

### Technical background

Melatonin (*N*-acetyl-5-methoxytriptamine) is a neurohormone produced primarily by the pineal gland of vertebrates. It displays a marked circadian pattern, with higher serum levels at night than during the day.

In humans, melatonin is involved in the regulation of biological rhythms, so its primary physiological function is the adaptation of the organism to the light-dark cycle. Due to this effect, exogenous melatonin administration is frequently used for the management of sleep disorders, including jet lag, insomnia, narcolepsy and sleep disorders in children. Analogously, the administration of melatonin is also useful for blind people and for shift workers, for helping in the synchronization of biological rhythms to the environment. Melatonin, as a natural, endogenous substance, is advantageous over conventional sedative treatments for sleep induction due to its excellent safety profile and the absence of dependence effects.

Furthermore, a variety of additional actions and biological functions have been reported for melatonin, which allows the potential for a range of therapeutic applications. Thus, for example, melatonin is a potent free radical scavenger and a general antioxidant and, therefore, it has been proposed for the treatment of cerebral infarction (Pei et al., Administration of melatonin after onset of ischemia reduces the volume of cerebral infarction in a rat middle cerebral artery occlusion stroke model, Stroke 2003, 34 (3), 770-5). Melatonin has also application for cancer prevention and as an adjuvant in cancer therapy (Li et al., Melatonin for the prevention and treatment of cancer, Oncotarget 2017, 8 (24), 39896-39921). Moreover, melatonin has immune-enhancing effect and thus may also be used for stimulating immune response in viral and bacterial infections (Carrillo-Vico et al., Melatonin: buffering the immune system, Int. J. Mol. Sci., 2013, 14 (4), 8638-8683). Melatonin has also neuroprotective effects, so it has been suggested for the inhibition of disease progression in Alzheimer's disease and other neurodegenerative disorders (Alghamdi B.S., The neuroprotective role of melatonin in neurological disorders, J. Neurosci. Res., 2018, 96 (7), 1136-1149).

So far, there are several medicinal products and dietary supplements available in the market that contain melatonin as active ingredient, mainly for the treatment of sleep disorders. For example, the medicine Circadin®, which is available as tablets containing 2 mg of melatonin, has been authorized by the European Medicines Agency for the treatment of primary insomnia. Other dietary supplements or nutraceuticals are also available, for example, in the form of pills, capsules, granules or solutions, optionally in combination with other active ingredients, for use as sleeping aids.

One drawback for the therapeutic use of melatonin is its low solubility in water. This fact hinders its use in the form of solutions, or as powders or granules for reconstitution, for example. Furthermore, the low solubility of melatonin in aqueous media may be partly responsible for the low and erratic bioavailability reported for this substance when administered in oral pharmaceutical forms, in general.

Several solutions have been proposed in the prior art in order to improve the solubility of melatonin in pharmaceutical dosage forms.

Thus, for example, in the international patent application WO99/47175-A1, it is disclosed that the preparation of inclusion complexes of melatonin in cyclodextrins results in an improvement of the solubility and bioavailability of melatonin. The melatonin-cyclodextrin inclusion complex may be prepared by known techniques, such as co-grinding, lyophilisation, granulation, spray-drying or precipitation, and may be used for preparing pharmaceutical compositions, for example, capsules, tablets or granulates.

In the international patent application WO2013/068565-A1, the preparation of a melatonin powder composition having improved solubility is disclosed, said composition comprising 35-90 wt% of melatonin, 5-60 wt% of at least one soluble excipient, such as mannitol or leucine, and 0.5-5 wt% of a surfactant, which is prepared by spray drying a solution comprising all the components.

In the article Johns et al., An intravenous injection of melatonin: formulation, stability, pharmacokinetics and pharmacodynamics, JAASP, 2012, 1 (1), 32-43, intravenous formulations of melatonin in water are disclosed, which comprise 2-hydroxypropyl-B-cyclodextrin and propylene glycol to increase the solubility of melatonin, since it is disclosed that melatonin is only slightly soluble in water.

The proposals disclosed so far in the art are technically burdensome and linked to specific formulations of melatonin. Therefore, there is the need of simple alternatives to provide suitable pharmaceutical formulations comprising melatonin which face the problem of the poor water solubility of this substance.

### Object of the invention

The object of the present invention is a process for obtaining compacted melatonin.

Another aspect of the invention is compacted melatonin obtainable with such process.

Another aspect of the invention is the use of compacted melatonin for the preparation of pharmaceutical compositions.

Another aspect of the invention is a pharmaceutical composition comprising compacted melatonin.

Another aspect of the invention a pharmaceutical composition comprising compacted melatonin for use in therapy.

### Description of the drawings

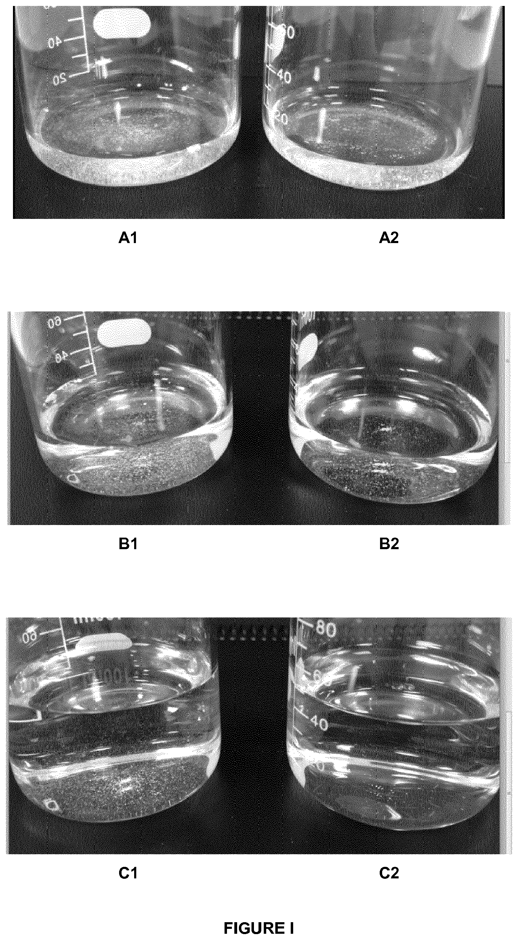

Figure 1 shows pictures with the result of three comparative dissolution assays using compacted and non-compacted melatonin. Pictures A1 and A2 correspond to an assay wherein 10 mg of non-compacted and compacted melatonin, respectively, were dissolved in 10 ml of water. Pictures B1-B2 and C1-C2 show the result of analogous assays using the same amount of melatonin, but dissolved in 25 and 50 ml of water, respectively.

### Detailed description of the invention

The object of the present invention is a process for preparing substantially pure compacted melatonin comprising roller compaction of melatonin.

The authors of the present invention have surprisingly found that, when substantially pure melatonin is subjected to roller compaction, the compacted melatonin thus obtained shows improved aqueous dissolution profile and, therefore, is advantageous for use in the preparation of pharmaceutical compositions.

Along the present description, as well as in the claims, the terms "a," "an," or "the" not only include aspects with one member (singular), but also include aspects with more than one member (plural).

The terms "about" or "approximately" referred to amounts, as used herein, are meant to include the exact amount and also a certain deviation around the stated amount, namely of ±5%.

The numerical ranges disclosed herein are meant to include any number falling within the ranges and also the lower and upper limits.

### Melatonin

Melatonin, also known as *N*-[2-(5-methoxy-1*H*-indol-3-yl)ethyl)acetamide or as *N*-acetyl-5-methoxy-tryptamine (CAS number 73-31-4) is a natural substance which can be found in vertebrates and also in plants.

Melatonin is commercially available from several sources, for example, melatonin can be obtained from the companies Santa Cruz Biotechnology, Cayman Chemical or Sigma Aldrich.

Melatonin can be prepared by known chemical synthetic methods, as disclosed, for example, in Prabhakar et al., Process research and development of melatonin, Org. Proc. Res. Dev., 1999, 3 (3), 155-160 or in Thomson et al., Efficient route to the pineal hormone melatonin by radical-based indole synthesis, Synth. Commun., 2003, 33, 3631-3641.

Furthermore, melatonin may also be obtained from natural sources, namely, from animal sources, for example, by extraction from bovine pineal glands, as disclosed in Lerner et al., Isolation of melatonin, the pineal-gland factor that lightens melanocytes, J. Am. Chem. Soc., 1958, 80, 2587. Melatonin can also be obtained by extraction from plants.

Melatonin from any source may be used as starting material for preparing compacted melatonin, of improved water solubility according to the present invention, provided that it has sufficient level of purity.

Non-compacted melatonin used as raw material for the present invention is substantially pure, i.e., it has typically a purity of at least 95%, preferably of at least 96%, more preferably of at least 97%, still more preferably of at least 98%, and still more preferably of at least 98.5%. The purity can advantageously be even of at least 99%, or of at least 99.5%, or about 100%. Accordingly, the expression "substantially pure" as used herein referred to melatonin, either to non-compacted melatonin or to compacted melatonin according to the present invention, means a degree of purity of at least 95%, preferably of at least 96%, more preferably of at least 97%, still more preferably of at least 98%, and still more preferably of at least 98.5%, also including a degree of purity of at least 99%, or of at least 99.5% or of about 100%.

The purity degree of melatonin can be determined using methods known in the art, for example, by HPLC.

The conventional, non-compacted, commercially available melatonin, as disclosed so far in the art, is reported to have poor water dissolution behaviour, i.e. low solubility and slow dissolution rate. Current commercial suppliers describe melatonin as only slightly soluble in water and recommend its dissolution in organic solvents, such as ethanol, DMSO and DMF.

Non-compacted, commercially available melatonin used for the preparation of compacted melatonin is typically in the form of powder or granules, with particle size generally of less than 1000 micron, or less than 700 microns, or less than 500 microns, or less than 300 microns, typically the particle size is in the range 100-300 microns. Particles of the desired size can be obtained by sieving, i.e., by passing through a sieve of desired mesh. Melatonin of any particle size is suitable to be used in the present invention.

### Compaction process

Commercially available melatonin, as disclosed above, is used as starting material to prepare compacted melatonin, according to the present invention.

The compaction process is performed using the roller compaction technology, which is well-known in the pharmaceutical technology field, namely, for the dry granulation of pharmaceutical powder mixtures. Such technology is widely described in different reference manuals in the art, for example, in R.W. Miller, Roller Compaction Technology, in: Handbook of Pharmaceutical Granulation Technology, Editor D.M. Parikh, Third Edition, Informa Healthcare USA, 2010, Chapter 8, 163-182. Briefly, in the roller compaction process, the powder being compacted is squeezed between two counter-rotating rolls to form a compressed sheet, which is subsequently milled into granules.

There are several parameters that can be adjusted in order to optimize the compaction process, namely, feed speed, roll speed, roll pressure, roll gap and mill speed.

As is well-known in the art, the feeding system in the roller compactor has the task of feeding the powder to be compacted to the rolls and must ensure a uniform flow of material in order to continuously fill the nip between the rolls as they rotate. It can be a gravity feeder, wherein the powder is fed vertically by gravity, or a force feeder, wherein the powder is pushed towards the rolls by one or several screws. Preferably, screw feeding is used in the present invention, and the feeder can be vertical, horizontal or inclined. In one preferred embodiment, the screw feeder is vertical and is situated in the upper part of the equipment, over the rolls. The screw feeder may be a hopper or analogous container containing an endless screw.

The feed speed can affect the properties of the compacted product, and thus it can be controlled by adjusting the feed screw r.p.m. The feed speed is preferably constant, and is typically comprised between about 5 and about 70 r.p.m. In a preferred embodiment of the invention, the feed speed is comprised between 10 and 30 r.p.m., more preferably is comprised between 10 r.p.m. and 20 r.p.m., and more preferably comprised between 10 r.p.m. and 15 r.p.m. In one embodiment, the feed speed is selected from about 10 r.p.m., about 11 r.p.m., about 12 r.p.m., about 13 r.p.m., about 14 r.p.m., about 15 r.p.m., about 16 r.p.m., about 17 r.p.m., about 18 r.p.m., about 19 r.p.m. and about 20 r.p.m.

Preferably, non-compacted melatonin raw material is sent for compaction into the screw feeder from another hopper or reservoir containing the product, for example, using vacuum for sucking the powder and sending it to the screw feeder in a controlled way. The sucking of the raw material and its delivery into the screw feeder can be controlled in an automatic way, for example, by placing a probe inside the feeder that activates the feeding of the raw material when necessary.

The roller unit consists of two equal diameter counter rotating rolls through which the powder is passed to get compacted. The two rolls can be mounted in horizontal, vertical or inclined position.

The roll speed can also be adjusted and may affect the characteristics of the compacted product. The roll speed typically is comprised between about 5 and about 35 r.p.m. In a preferred embodiment of the invention the roll speed is comprised between 5 and 20 r.p.m., more preferably comprised between 5 and 15 r.p.m., and still more preferably between 7 and 10 r.p.m.

Another adjustable parameter is the roll gap, i.e., the distance between the two rolls. It typically ranges from about 0.2 mm to about 2 mm, preferably from about 0.5 mm to about 1.6 mm, more preferably from about 1.0 mm to about 1.5 mm. In particularly preferred embodiments of the invention, the roll gap is selected from about 1.0 mm, about 1.1 mm, about 1.2 mm, about 1.3 mm, about 1.4 mm and about 1.5 mm. In a particularly preferred embodiment, the roll gap is about 1.3 mm.

Generally, one roll is fixed, while the other is movable and exerts pressure on the fixed roll by means of a hydraulic pressure control system. The horizontal pressure/force between rolls can be also adjusted and may also have influence on the properties of the end-product. The roll pressure/force typically ranges from about 4000 to about 18000 Kgf, preferably from about 5000 to about 16000 Kgf. In an embodiment of the invention, the pressure/force between rolls ranges from 12000 to 17000 Kgf, preferably from 13000 to 16000 Kgf, more preferably from 14000 to 15000 Kgf.

The roll surface may be either smooth or non-smooth, i.e., may have rough pattern surface, for example, fluted, grooved or knurled surface. The roll surface of the two rolls may be identical or different.

In one embodiment of the invention, both rolls have smooth surface. In another embodiment of the invention, one roll has a smooth surface and the other has a non-smooth surface, for example, fluted, grooved or knurled surface.

Once melatonin has been compacted by passing through the two rolls, it is transformed in a compressed sheet or ribbon, which is subsequently sent to the granulation module. Such granulation module comprises one or more mills which break the compacted ribbon into compacted granules which are sieved through the desired mesh size to obtain granules of the desired particle size. In this step of the compaction process, the speed of the granulation mill can also be adjusted and is typically comprised in the range of from about 50 to about 500 r.p.m, preferably from 55 to 200 r.p.m., more preferably from 60 to 100 r.p.m. In particularly preferred embodiments, the speed of the granulation mill is selected from about 60 r.p.m., about 70 r.p.m., about 80 r.p.m., about 90 r.p.m. and about 100 r.p.m. In a particularly preferred embodiment, the speed of the granulation mill is about 70 r.p.m.

In one particularly preferred embodiment of the invention, melatonin is subjected to roller compaction using the following parameters:
- Feed speed is comprised between 10 r.p.m. and 15 r.p.m., preferably is selected from about 10 r.p.m., about 11 r.p.m., about 12 r.p.m., about 13 r.p.m., about 14 r.p.m. and about 15 r.p.m., and more preferably is about 11 r.p.m.
- Roll speed is comprised between 5 r.p.m. and 15 r.p.m., preferably is comprised between 7 r.p.m. and 10 r.p.m.
- Roll gap is comprised between 1.0 mm and 1.5 mm, preferably is selected from about 1.0 mm, about 1.1 mm, about 1.2 mm, about 1.3 mm, about 1.4 mm, and about 1.5 mm, and more preferably is about 1.3 mm.
- Roll pressure/force between the rolls is comprised between 13000 Kgf and 16000 Kgf, preferably between 14000 Kgf and 15000 Kgf.
wherein the speed of the granulation mill is preferably comprised between 60 r.p.m. and 100 r.p.m., preferably is selected from about 60 r.p.m., about 70 r.p.m., about 80 r.p.m., about 90 r.p.m., and about 100 r.p.m., more preferably is about 70 r.p.m. Preferably one roll has smooth surface and the other has a non-smooth surface.

Typically, the roller compaction technology is used in the pharmaceutical field as a step in the preparation of solid pharmaceutical dosage forms, as an alternative to the wet granulation technology for preparing granules containing the active ingredient together with excipients. Therefore, typical roller compaction formulations include the active ingredient together with pharmaceutical excipients usually selected from fillers, diluents, binders, disintegrants, lubricants, glidants, surfactants and mixtures thereof, as disclosed in R.W. Miller *op. cit.* Conversely, the compaction of active ingredients alone, without the aid of adequate excipients, is regarded as problematic, and is generally considered unsuitable in order to prepare final pharmaceutical formulations. Consequently, this approach is rarely disclosed in the art. Furthermore, the compaction of powders using the roller compaction methodology is generally acknowledged to involve a mixture of bonding mechanisms, through consecutive processes of rearrangement, deformation, fragmentation and bonding of the particles. Such process is highly dependent on the nature of the powder particles being compressed and is therefore highly unpredictable.

Moreover, the positive effect of roller compaction on the dissolution behaviour of melatonin, as found in the present invention, had not been disclosed before in the art for other substances. On the contrary, roller compaction is generally believed to adversely affect the dissolution profile of the compacted substance precisely because the compaction process is expected to reinforce the bonding mechanisms between the substances. Roller compaction has only occasionally been related to an improvement in the disintegration or dissolution rate of compacted pharmaceutical compositions, but only as long as such compositions included specific excipients, namely, disintegrants or dissolution enhancers, as additional components of the powder mixture being compressed.

The authors of the present invention have surprisingly found that particles of melatonin can be successfully compacted on their own, using the roller compaction methodology, without adding any auxiliary excipient. Furthermore, unexpectedly, it was found that the compacted, substantially pure melatonin obtained after the roller compaction process showed improved solubility and dissolution rate compared to non-compacted melatonin, irrespective of the particle size.

As can be seen in the comparative dissolution assay of Example 1, when compacted melatonin according to the present invention is used (assay C2) a clear solution of melatonin in water is obtained, while when commercial melatonin was used (assay C1) melatonin was not completely dissolved and a dispersion of solid, not-dissolved melatonin was obtained instead (see Figure 1).

Therefore, another aspect of the invention is compacted melatonin which is obtainable by a roller compaction process, as disclosed above.

Remarkably, after the compaction process, melatonin maintains its chemical integrity, so there is neither loss in purity nor increase in impurity occurrence. Therefore, the compaction process according to the present invention allows the preparation of substantially pure compacted melatonin, with improved water solubility, which has substantially the same purity degree as the starting material.

The compacted melatonin according to the present invention typically has a bulk density equal to or higher than 0.22 g/ml, preferably higher than 0.24 g/ml, more preferably higher than 0.26 g/ml, still more preferably higher than 0.28 g/ml, and still more preferably higher than 0.30 g/ml. Typically, the bulk density of compacted melatonin according to the present invention is comprised between about 0.3 g/ml and about 0.55 g/ml, preferably comprised between 0.30 g/ml and 0.40 g/ml.

The bulk density, as used in the present description, is defined as the ratio of the mass to the volume of an untapped powder sample, as "poured". The bulk density is given in grams per millilitre (g/ml) and is typically calculated by "pouring" a known mass of powder into a graduated cylinder. Bulk density can be measured according to the method disclosed in the European Pharmacopoeia section 2.9.34 (*Bulk density and tapped density of powders),* for example, using the PT-SV100 Scott Volumeter (Pharma Test Apparatebau AG, Germany).

The size of the particles of compacted melatonin can be adjusted, for example, by milling, if necessary, and sieving, using a sieve of suitable mesh size to obtain particles of the desired size, namely, under a specific size determined by the sieve opening. Typically, the mesh size of the sieve is adjusted to obtain particles of less than 1000 microns, preferably less than 900 microns, more preferably less than 800 microns, still more preferably less than 700 microns, still more preferably less than 600 microns, still more preferably less than 500 microns, and still more preferably less than 400 microns.

It was found that compacted melatonin showed improved water solubility and/or dissolution rate compared to non-compacted melatonin, irrespective of the particle size. Surprisingly, better dissolution was achieved with compacted melatonin even if the particle size was the same or even larger than in the starting non-compacted melatonin. This fact was surprising because it is well-known in the art that, for a given substance, there is a direct effect of the particle size on the dissolution, so, generally, the smaller the particle size, the higher and faster the dissolution.

### Pharmaceutical compositions

The melatonin obtained according to the method of the present invention, having improved solubility in water, can be advantageously used in the preparation of pharmaceutical compositions.

It is well-known in the art that, for poorly water-soluble and/or slow dissolving substances, dissolution may be the rate-limiting step in absorption and may influence drug bioavailability. Indeed, melatonin has been reported to have low and erratic bioavailability, therefore, using melatonin having better dissolution profile can contribute to improve its bioavailability.

Furthermore, the improvement in the aqueous dissolution profile of melatonin may also be advantageous for preparing certain pharmaceutical dosage forms, particularly, liquid or semi-solid compositions where melatonin is dissolved in an aqueous vehicle, and solid forms that are intended to be reconstituted, i.e., dissolved in water previously to the administration.

Therefore, another aspect of the invention is the use of compacted melatonin for the preparation of a pharmaceutical composition.

Another further aspect of the invention is a pharmaceutical composition comprising compacted melatonin and at least one pharmaceutically acceptable excipient and/or vehicle.

Another aspect of the invention is a process for preparing a pharmaceutical composition comprising melatonin and at least one pharmaceutically acceptable excipient and/or vehicle comprising a step of previously compacting melatonin by roller compaction.

It is understood that melatonin is the active ingredient of the pharmaceutical composition. Optionally, the composition can contain one or more additional active ingredients to reinforce the desired therapeutic activity of the composition.

Typically, the pharmaceutical composition comprising compacted melatonin as active ingredient, according to the present invention can be any conventional formulation which can be prepared using methods that are well known to the person skilled in pharmaceutical formulation, as those disclosed in handbooks of pharmaceutical technology, for example, in the book J.P Remington and A. R. Genaro, Remington The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 4th edition, Churchill Livingstone Elsevier, 2013 [ISBN: 978-0-7020-4290-4], or in the book A.K. Dash, S. Singh and J. Tolman, Pharmaceutics. Basic principles and application to pharmacy practice, Academic Press, Elsevier, 2014 [ISBN: 978-0-12-386890-9].

The excipients suitable to be used in the pharmaceutical compositions of the present invention are also well known to those skilled in pharmaceutical technology and are described, for example, in the book R.C. Rowe, P.J. Sheskey and P.J. Weller, Handbook of Pharmaceutical Excipients, Sixth Edition, Pharmaceutical Press, 2009.

Suitable formulations for compacted melatonin include not only oral forms, but also injectable and topical dosage forms.

Oral pharmaceutical compositions may be solid, such as tablets, capsules, powders or granules; or liquid, such as solutions, suspensions or syrups.

Tablets comprising compacted melatonin can be formulated using standard procedures, well known in the art. Preferably, tablets are prepared by direct compression, i.e., compacted melatonin is mixed with suitable excipients and compressed without any previous granulation process. Alternatively, compacted melatonin can be previously granulated with additional excipients, either by wet granulation, fluid-bed granulation or dry granulation, and then optionally mixed with extra-granular excipients and compressed into tablets. Excipients typically used in the formulation of tablets are diluents, binding agents, glidants, lubricants, disintegrating agents, and mixtures thereof. Other additional excipients include sweetening agents, flavouring agents and colouring agents, for example.

Suitable diluents include calcium carbonate, calcium phosphate, calcium sulfate, cellulose acetate, dextrates, dextrins, dextrose, ethylcellulose, fructose, glyceryl palmitostearate, isomalt, kaolin, lactitol, lactose, magnesium carbonate, magnesium oxide, maltodextrins, maltose, mannitol, microcrystalline or powdered cellulose, pregelatinized starch, starch, sodium carbonate, sodium chloride, sorbitol and sucrose, among others, and mixtures thereof.

Suitable binding agents include acacia, agar, cellulose acetate phthalate, copovidone, dextrates, dextrin, dextrose, ethylcellulose, gelatin, glyceryl behenate, guar gum, hydroxyethyl cellulose, hydroxyethylmethyl cellulose, hydroxypropyl cellulose, hydroxypropylmethyl cellulose, methylcellulose, maltodextrin, microcrystalline cellulose, povidone, pregelatinized starch, sodium carboxymethylcellulose, starch, stearic acid and sucrose, among others, and mixtures thereof.

Suitable glidants include powdered cellulose, colloidal silicon dioxide, magnesium oxide, magnesium silicate, magnesium trisilicate, silicon dioxide, and talc, among others, and mixtures thereof.

Suitable lubricants include calcium stearate, glyceryl behenate, glyceryl palmitostearate, hydrogenated castor oil, magnesium stearate, myristic acid, palmitic acid, polyethylene glycol, sodium benzoate, sodium lauryl sulfate, sodium stearyl fumarate, stearic acid, and talc, among others, and mixtures thereof.

Suitable disintegrants include alginic acid, crospovidone, sodium croscarmellose, sodium starch glycolate, starch, and low-substituted hydroxypropyl cellulose, among others, and mixtures thereof.

Furthermore, tablets may be formulated either as conventional compressed tablets, or in other forms, for example, buccal, sublingual, chewable, sustained-release or orally-disintegrating tablets, by selecting suitable excipients and manufacturing processes, as are well known in the art.

Capsules, as is well known in the pharmaceutical field, are solid dosage forms in which the drug substance is enclosed in either a hard or soft, soluble container or shell. The raw materials used in the manufacture of the shell are similar for both hard and soft gelatin capsules, while the proportions vary. The major component of a capsule shell is gelatin; other components include water, plasticizers, such as glycerine, sorbitol or propylene glycol, colorants, opacifiers, such as titanium dioxide, and preservatives. Hypromellose can alternatively be used as capsule shell material for hard capsules.

Hard capsules consist of two sections, one slipping over the other and thus enclosing the drug formulation, which is generally in solid form, as powder or granulate, or occasionally in liquid or semi-liquid form, while in soft gelatin capsules, also referred to as softgels, a continuous, one-piece, hermetically sealed gelatin shell surrounds the drug formulation, which is generally in liquid, suspension or semisolid form.

The powders or granules included within hard capsules comprise the active ingredient and typically at least one pharmaceutically acceptable excipient, generally selected from diluents, lubricants and glidants, and mixtures thereof, which are well-known in the art, for example, those disclosed above for the formulation of tablets.

The term "powders" typically refers to intimate mixtures of finely divided active ingredient, optionally with one or more excipients. The active ingredient and the excipient(s) are mixed to obtain an homogeneous mixture, for example using trituration, spatulation, sifting or tumbling procedures, which are well-known in the art.

The term "granules" typically refers to larger particles, either containing the active ingredient alone, or, more frequently, aggregations of active ingredient particles with additional excipients, typically prepared by dry granulation, wet granulation or fluid-bed granulation procedures, which are also well known in the art.

The fill compositions for soft gelatin capsules generally comprise the active ingredient, i.e., melatonin, dissolved or dispersed in a liquid vehicle. The vehicle is typically an oily liquid, for example, a vegetable oil, such as peanut oil, castor oil, olive oil, rapeseed oil, corn oil, sesame oil, sunflower oil or soybean oil, among others, or medium chain triglycerides (MCT), or mixtures thereof. Other liquid, non-oily, vehicles can also be used, for example, polyethylene glycols, isopropyl alcohol, propylene glycol, glycerol, polyglycerols, triacetin, glyceryl esters, sorbitan esters, sugar esters and polyglyceryl esters, among others, or mixtures thereof. The composition generally additionally comprises other excipients, acting as suspending agents and/or viscosity modifying agents, for example, hydrogenated vegetable oils, waxes, or ethylcellulose for oily vehicles and solid glycol esters for non-oily vehicles. Surfactants such as lecithin or polysorbate 80 may also be added to improve the dispersion of the suspended drug particles. Alternatively, the fill composition may be in the form of self-emulsifying lipophilic systems or microemulsion pre-concentrates, which typically comprise a lipophilic solvent, such as a vegetable oil or a medium chain triglyceride, and a surfactant, and optionally a co-solvent, such as ethanol or propyleneglycol.

Powders or granules for oral administration may be directly administered to the oral cavity to be swallowed or either they can be previously dissolved or dispersed in water or in other suitable liquid before being ingested. They may also be effervescent powders or granules.

The formulation and preparation of powders or granules for oral administration is analogous to the formulation and preparation of powders or granules for preparing tablets or for filling capsules, as discussed above.

They typically comprise the active ingredient, i.e., melatonin, and at least one pharmaceutically acceptable excipient, generally selected from diluents, lubricants, glidants, binders, disintegrating agents, and mixtures thereof, which are well-known in the art, for example, those disclosed above for the formulation of tablets. They may contain also additional excipients, such as colouring and flavouring substances. Furthermore, as is well-known in the art, effervescent powders additionally contain acid substances, for example, citric acid, and carbonates or hydrogen carbonates which react rapidly and effervesce releasing carbon dioxide.

Powders or granules for oral administration are presented as single-dose or multi-dose preparations in suitable containers, for example, bulk containers provided with a measuring device for multi-dose preparations, or mono-dose sachets for single-dose preparations. Those sachets can be made of paper or either of aluminium or plastic laminates.

In one embodiment, the pharmaceutical composition comprising compacted melatonin and at least one pharmaceutically acceptable excipient is a solid form for oral administration selected from the group consisting of tablets, hard capsules, soft capsules, powders and granules.

Suitable liquid oral dosage forms typically include solutions, suspensions and syrups.

Oral solutions contain melatonin active substance dissolved in a vehicle, which is generally water, optionally, with additional co-cosolvents. Syrups are oral aqueous solutions containing high concentrations of sucrose or other sugars. Sugar-free syrups are obtained by replacing sucrose with hydrogenated glucose, mannitol, sorbitol or xylitol, for example. Oral suspensions contain melatonin dispersed in a liquid medium. Suitable excipients included in solutions and/or suspension are, for example, solubilizers, stabilizers, buffers, antioxidants, preservatives, flavouring agents, colouring agents, and sweetening agents, for example.

Suitable co-solvents include ethanol, propylene glycol, polyethylene glycol 300 or 400, sorbitol and glycerine, among others, and mixtures thereof.

Suitable solubilizing agents include cyclodextrins and surfactants, such as sorbitan esters, polyoxyethylene castor oil derivatives, polysorbates, sodium oleate, potassium oleate, or polyoxyethylene stearate, among others, and mixture thereof.

Suitable preservatives include ascorbic acid, sorbic acid, benzalkonium chloride, benzyl alcohol, bronopol, parabens, sodium benzoate, sodium propionate, or thimerosal, among others, and mixtures thereof.

Suitable antioxidants include ascorbic acid, sodium sulphite, sodium bisulphite and sodium metabisulfite, among others, and mixtures thereof.

Suitable viscosity modifiers include acacia, alginic acid, bentonite, carbomers, carrageenan, gelatin, glycerin, hydroxyethyl cellulose, hydroxyethylmethyl cellulose, hydroxypropyl cellulose, maltodextrin, polyvinyl alcohol, sodium alginate, tragacanth, or xanthan gum, gellan gum, guar gum, among others and mixtures thereof.

Suitable suspending agents include xanthan gum, guar gum, alginic acid, bentonite, carbomers, sodium or calcium carboxymethylcellulose, hydroxyethyl cellulose, hydroxypropyl cellulose, hydroxypropylmethyl cellulose, hydroxypropyl alginate, microcrystalline or powdered cellulose, anhydrous colloidal silica, dextrins, gelatins, kaolin, magnesium aluminium silicate, maltitol, povidone, sorbitan esters, or tragacanth, among others, and mixtures thereof.

Suitable flavouring agents include maltol, vanillin, ethyl vanillin, menthol, citric acid, fumaric acid, ethyl maltol, tartaric acid, peppermint, artificial or natural fruit aromas, among others, and mixtures thereof.

Suitable sweetening agents include sorbitol, maltitol, mannitol, dextrose, isomalt, maltose, xylitol, saccharin, sucrose, sucralose, aspartame, acesulfame potassium or trehalose, among others, and mixtures thereof.

Typically, such liquid dosage forms for oral use may be supplied as multi-dose or as single-dose preparations. Each dose from a multi-dose container is generally administered using a device suitable for measuring the prescribed volume. The measuring device may be, for example, a spoon, a cup, an oral syringe, or a dropper.

In one embodiment, the pharmaceutical composition comprising compacted melatonin and at least one pharmaceutically acceptable excipient is a liquid formulation for oral administration selected from the group consisting of solutions, suspensions and syrups. Preferably the oral liquid composition is a solution.

Compacted melatonin can also be formulated as a parenteral composition, namely, for intravenous or intramuscular administration. Parenteral or injectable compositions, as is well-known in the art, are sterile formulations in the form of solution, emulsion or suspension, prepared by dissolution, emulsification or suspension of melatonin active ingredient in water for injection, or, alternatively, in other vehicles. Alternatively, the injectable composition may be in the form of powder, for preparing the solution immediately before the administration by dissolving the powder in water for injection.

Suitable excipients that can be added to parenteral formulations include antimicrobials, such as benzalkonium chloride, benzyl alcohol, chlorobutanol, metacresol, parabens, phenol or thimerosal, among others, and mixtures thereof; antioxidants, such as ascorbic acid, cysteine, monothioglycerol, sodium bisulfite, sodium metabisulfite, tocopherols, among others, and mixtures thereof; buffers, such as acetates, citrates or phosphates, among others; chelating agents, such as salts of EDTA; solubilizing agents, such as ethyl alcohol, glycerin, polyethylene glycol, or propylene glycol, among others, and mixtures thereof; surfactants, such as polyoxyethylene or sorbitan monooleate, among others; and tonicity-adjusting agents, such as dextrose or sodium chloride, among others, or mixtures thereof; among others, and mixtures thereof.

In one embodiment, the pharmaceutical composition comprising compacted melatonin and at least one pharmaceutically acceptable excipient is an injectable composition.

Compacted melatonin can also be formulated as a topical composition, namely, in liquid or semisolid form. Liquid topical compositions include lotions, liniments and tinctures, and they are typically prepared by dissolving or dispersing melatonin in a suitable carrier such as, for example, water, alcohols, glycols, or mixtures thereof. Topical liquid compositions may be directly applied on the area of skin or mucosal tissue to be treated or, alternatively, they can be used to impregnate a dressing or bandage to be applied on the treated area.

Semisolid topical compositions include creams, gels, ointments and pastes. Typically, they are prepared by dissolving or suspending melatonin in a suitable pharmaceutically acceptable carrier. This carrier is selected from water, a non-aqueous water miscible carrier, such as for example ethanol or isopropanol, and a non-aqueous water-immiscible carrier, such as for example paraffin oil. Preferably, semisolid topical compositions for melatonin are in the form of creams. As is well-known in the art, creams are semisolid emulsions, which are classified as oil-in-water (o/w) and water-in-oil (w/o) types depending on whether the continuous phase is oil or water. Typically, such semisolid compositions for topical administration contain a pharmaceutically acceptable excipient such as, for example, surfactant and emulsifier agents, lipidic and emollient compounds, consistency factors and thickening agents, stabilizers, hydrotropes, preservative agents, essences, colouring agents, silicone compounds, fats, waxes, lecithins, phospholipids, UV sun protection factors, or mixtures thereof.

Suitable topical compositions also include ophthalmic solutions, which are intended for application to the conjunctiva, conjunctival sac or eyelids and may be in the form of aqueous solutions, suspensions, emulsions, or reconstitutable powders. The formulation of ophthalmic compositions is well known in the art, and common excipients employed include the vehicle, which is typically water for injection, buffering agents, preservatives, tonicity agents, viscosity modifiers, surfactants and stabilizers.

In one embodiment, the pharmaceutical composition comprising compacted melatonin and at least one pharmaceutically acceptable excipient is a topical composition selected from the group consisting of lotions, liniments, tinctures, creams, gels, ointments, pastes, and ophthalmic solutions, preferably selected from lotions, creams and ophthalmic solutions.

The dose of melatonin contained in each unit dose may widely vary depending on the dosage form, the route of administration and the therapeutic indication. Therefore, the unit dose of melatonin may be comprised in the range 0.1-30 mg, for example selected from the following doses: about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 mg of melatonin per unit dose.

Alternatively, compacted melatonin may also be added to nutraceutical or dietary supplement formulations, taking advantage of its better solubility profile, for example, in functional drinks wherein melatonin, as active ingredient, is contained in a drink-based formulation, or in a solid formulation intended to be dissolved in water, optionally, in combination with other active ingredients. Other suitable formulations for compacted melatonin are, for example, infusion formulations, where melatonin can be added to medicinal herbs, taking advantage of its improved water solubility.

### Use

The pharmaceutical dosage forms prepared with the more water-soluble compacted melatonin as active ingredient are advantageous because their manufacturing process may be easier, when it comprises steps where melatonin has to be dissolved, and also because the improved solubility may facilitate patient's medication intake. Furthermore, the improved solubility behaviour of compacted melatonin may also contribute to provide better bioavailability of melatonin, which can be advantageous also in terms of therapeutic efficacy.

Therefore, another aspect of the present invention relates to a pharmaceutical composition comprising compacted melatonin for use in therapy.

Another aspect of the invention is a method of treating a disease or condition susceptible to treatment with melatonin in a subject, the method comprising administering a pharmaceutical composition comprising compacted melatonin to the subject.

The main therapeutic indications of melatonin are those related to the management of sleep disorders, including insomnia, jet lag, and sleep disorders in children, in the elderly and in shift workers, for example.

Other described indications for melatonin are, for example, the prevention and/or treatment of cancer (such as breast cancer, prostate cancer or colorectal cancer, among others), epilepsy or depression; the stimulation of the recovery after cerebral infarction, myocardial ischemia-reperfusion, and myocardial infarction; the stimulation of immune response in viral and bacterial infections; the inhibition of disease progression in Alzheimer's disease and other neurodegenerative disorders; the treatment of gastric ulcers, pancreatitis, irritable bowel syndrome, ulcerative colitis and diarrhea; or the treatment of drug-induced diseases, such as acute renal injury or cardiotoxicity.

Melatonin may also be used topically in dermatology, for example, for the treatment of UV-induced erythema, or in ophthalmology, for example, for the treatment of glaucoma and keratitis.

The terms "treatment" or "treating" as used herein have their conventional meaning and typically refer to the administration of the pharmaceutical composition with the purpose of attenuating, alleviating, minimising, inhibiting the progression, or suppressing the symptoms of the treated disease or condition, or for stimulating the recovery after said disease or condition. Furthermore, the treatment is also meant to include the preventive aspect, i.e., the administration of the melatonin containing pharmaceutical composition to a subject suspected or prone to suffer from a specific disease or condition in order to prevent the onset of such disease or condition.

The pharmaceutical composition comprising compacted melatonin for use in therapy, according to this aspect of the invention, is meant therefore to include the treatment and/or prevention of any disease or condition susceptible to be treated or prevented with melatonin.

For use in those indications, melatonin may be administered alone, or as adjuvant to other therapies.

The therapeutic applications of melatonin are described, for example, in the book Melatonin in the promotion of health, R Ross Watson, Ed., CRC Press, Second Edition, 2012, or in the book Melatonin and melatonergic drugs in clinical practice, V. Srinivasan, A. Brzezinski, S. Oter and S. D. Shillcutt, Eds., 2014, Springer, or in the review article Malhotra et al., The therapeutic potential of melatonin: a review of the science, MedGenMed 2004, 6 (2).

The therapeutically effective dose of melatonin widely varies depending on the therapeutic indication, the route of administration, the severity of the condition, and the age of the patient. So, the recommended dose may typically range from 0.5 to 50 mg/day. The expert practitioner will have no difficulty in selecting the most appropriate dosage in each case.

The particular and preferred embodiments of the pharmaceutical composition regarding its use in therapy are the same as described above in the previous section.

Next, some examples are provided with the purpose of illustrating the invention, but not limiting it.

### Examples

### Example 1.- Preparation of compacted melatonin

For the preparation of compacted melatonin, commercially available melatonin (Cayman Chemical) was used. The melatonin raw material was a granular powder, of bulk density in the range 0.25-0.30 g/ml and particle size of less than 400 microns.

The purity of melatonin before and after the compaction process was assessed by means of HPLC. The HPLC specifications used are as follows:

| | |
|---|---|
| Equipment: | 1220 Infinity LC (Agilent Technologies). |
| Column: | ACE3 C18 PFP (150 x 4.6 mm) |
| Flow rate: | 1 ml/min |
| Injection volume: | 10 µl |
| Detector: | 222 nm |
| Run time: | 10 minutes |
| Solvent: | water:methanol (45:55). Isocratic elution |
| Retention time: | 3.75 |

Both the original melatonin and the compacted melatonin obtained after the compaction process had chemical purity greater than 99%, determined by HPLC.

For the compaction process, a roller compactor equipment was used (Bonals BC-150/75V), with vacuum system for feeding the raw material into the screw feeding system. The roller compactor had one roll with smooth surface and one roll with knurled surface. The dimensions of both rolls were 10.5 cm diameter and 7.4 cm width.

The roll speed in the roller compactor was about 7-10 r.p.m. Screw feeding speed was 11 r.p.m., roll force was set to 14000-15000 Kgf and the roll gap was fixed to 1.3 mm.

The compactor was connected to a rotor granulator for milling the outcoming compacted plaque. The granulation speed was fixed to 70 r.p.m. and the obtained granulate was passed through a sieve of 400 micron opening.

The compacted melatonin obtained had a bulk density of 0.34 g/ml and 100% of the particles had a size of less than 400 micron.

### Example 2.- Solubility of compacted melatonin

The water solubility of non-compacted melatonin (starting material used for compaction in Example 1) and the solubility of the compacted melatonin prepared in Example 1 were compared.

Three samples of 10 mg each of non-compacted melatonin and three samples of 10 mg each of compacted melatonin were introduced in separate beakers and tap water at 22 °C was added to each beaker to a volume of 10, 25 or 50 ml (assays A, B and C, respectively). Each tested mixture was sonicated for 5 minutes and subsequently subjected to magnetic stirring at 200 rpm for 2 more minutes.

The solubility tests performed are summarized in the following table:

| **Assay** | **Non-compacted melatonin** | **Compacted-melatonin** |
|---|---|---|
| A | A1 | A2 |
| (10 mg / 10 ml) | (partially dissolved) | (partially dissolved) |
| B | B1 | B2 |
| (10 mg / 25 ml) | (partially dissolved) | (partially dissolved) |
| C | C1 | C2 |
| (10 mg / 50 ml) | (partially dissolved) | (totally dissolved) |

In each case, the assays with compacted melatonin provided higher dissolution rate, as could be determined by ocular inspection. Figure 1 shows pictures with the result of each of the above dissolution assays.

In assay C2, melatonin was completely dissolved, while in the assay in the same conditions with non-compacted melatonin (C1) some undissolved solid remained still visible.

Using HPLC analysis, it was found that in assay C2 the 100% of melatonin was available in the solution, whereas in assay C1 only the 79 % of melatonin was solubilized, i.e., the 21% remained as solid, not dissolved.

## Claims

1. A process for preparing substantially pure compacted melatonin comprising roller compaction of melatonin.

2. Process according to claim 1, **characterized in that** a roller compactor is used comprising a screw feeder and the feed speed is adjusted to a value comprised between 10 r.p.m. and 30 r.p.m.

3. Process according to claim 1 or 2, **characterized in that** a vacuum system is employed for feeding melatonin raw material into the screw feeder.

4. Process according to any one of claims 1 to 3, **characterized in that** the roll speed is adjusted to a value comprised between 5 r.p.m. and 15 r.p.m.

5. Process according to any one of claims 1 to 4, **characterized in that** the roll gap is adjusted to a value comprised between 1.0 mm and 1.5 mm.

6. Process according to any one of claims 1 to 5, **characterized in that** the roll force is adjusted to a value comprised between 13000 Kgf and 16000 Kgf.

7. Process according to any one of claims 1 to 6, **characterized in that** one of the rolls has a smooth surface and the other has a non-smooth surface.

8. Substantially pure, compacted melatonin which is obtainable by a process according to any one of claims 1 to 7.

9. Compacted melatonin according to claim 8, **characterized in that** it has a particle size of less than 1 mm.

10. Use of compacted melatonin according to claims 8 or 9 for the preparation of a pharmaceutical composition.

11. Pharmaceutical composition comprising compacted melatonin according to claims 8 or 9 and at least one pharmaceutically acceptable excipient and/or vehicle.

12. Pharmaceutical composition according to claim 11, **characterized in that** it is an oral pharmaceutical composition selected from tablets, capsules, powders or granules, solutions, suspensions and syrups.

13. Pharmaceutical composition according to claim 11, **characterized in that** it is a topical pharmaceutical composition selected from lotions, creams and ophthalmic solutions.

14. Pharmaceutical composition according to claim 11, **characterized in that** it is an injectable composition.

15. Pharmaceutical composition according to any one of claims 11 to 14 for use in therapy.
